# EUROPÄISCHE PATENTSCHRIFT

(11) **EP 2 768 327 B1**
(45) Veröffentlichungstag und Bekanntmachung des Hinweises auf die Patenterteilung: **02.03.2016**
(21) Anmeldenummer: 12778091.4
(22) Anmeldetag: 18.10.2012
(51) Int. Cl.: A61M 15/06, A24F 47/00, A61M 11/04

(54) **INHALATORKOMPONENTE**
INHALER COMPONENT
COMPOSANT D'INHALATEUR

(30) Priorität: 21.10.2011 AT 15432011
(43) Veröffentlichungstag der Anmeldung: 27.08.2014
(60) Teilanmeldung: 16151458.3
(73) Patentinhaber: Batmark Limited, London WC2R 2PG (GB)
(72) Erfinder: BUCHBERGER, Helmut, 4482 Ennsdorf (AT)
(74) Vertreter: Schrell, Andreas
(86) Internationale Anmeldenummer: PCT/EP2012/070647
(87) Internationale Veröffentlichungsnummer: WO 2013/057185

(56) Entgegenhaltungen:
- EP-A1- 2 340 729
- WO-A1-2010/045671
- US-A- 6 155 268
- US-A1- 2004 129 793

## Beschreibung

Die Erfindung betrifft eine Inhalatorkomponente für die Bildung eines Dampf-Luft-Gemisches oder/und Kondensationsaerosols durch Verdampfung eines flüssigen Materials und gegebenenfalls Kondensation des gebildeten Dampfes, umfassend:
ein Gehäuse;
ein elektrisches Heizelement zur Verdampfung einer Portion des flüssigen Materials;
einen Docht mit einer Kapillarstruktur, welcher Docht mit dem Heizelement einen Verbund bildet und das Heizelement selbsttätig mit dem flüssigen Material versorgt;
eine Trägerplatte, vorzugsweise Leiterplatte, welche den Verbund trägt und auf welcher das Heizelement elektrisch kontaktiert ist;
einen zumindest zum Teil durch die Trägerplatte gebildeten Kapillarspalt zur selbsttätigen Versorgung des Verbundes mit dem flüssigen Material, indem ein Endabschnitt des Dochts in den Kapillarspalt ragt.

### Begriffsdefinition:

In der gegenständlichen Patentanmeldung bezieht sich der Begriff "Inhalator" auf medizinische wie nicht-medizinische Inhalatoren. Der Begriff bezieht sich ferner auf Inhalatoren zur Verabreichung von Arzneimitteln und solchen Stoffen, welche nicht als Arzneimittel deklariert sind. Der Begriff bezieht sich außerdem auf Rauchartikel und Zigarettenersatz-Artikel, wie sie beispielsweise in der Europäische Patentklasse A24F47/00B enthalten sind, soweit diese dazu bestimmt sind, dem Benutzer ein Dampf-Luft-Gemisch oder/und Kondensationsaerosol darzureichen. Der Begriff "Inhalator" soll auch keine Einschränkungen dahingehend machen, wie das gebildete Dampf-Luft-Gemisch oder/und Kondensationsaerosol dem Benutzer bzw. dessen Körper zugeführt wird. Das Dampf-Luft-Gemisch oder/und Kondensationsaerosol kann in die Lunge inhaliert werden, oder aber auch nur der Mundhöhle zugeführt werden - ohne Inhalation in die Lunge.

Als "Kapillarspalt" gelte ein jeder Spalt, welcher allein aufgrund der Kapillarwirkung seiner Begrenzungswände einen Flüssigkeitstransport bewirkt. Dochte, ummantelte Dochte oder mit Dochtmaterial befüllte Kanäle sind keine Kapillarspalten.

Die Verwendung des Singulars "Verbund" schließt das Vorhandensein mehrerer Verbunde nicht aus. Die Erfindung schließt Anordungen mit mehreren Verbunden explizit ein.

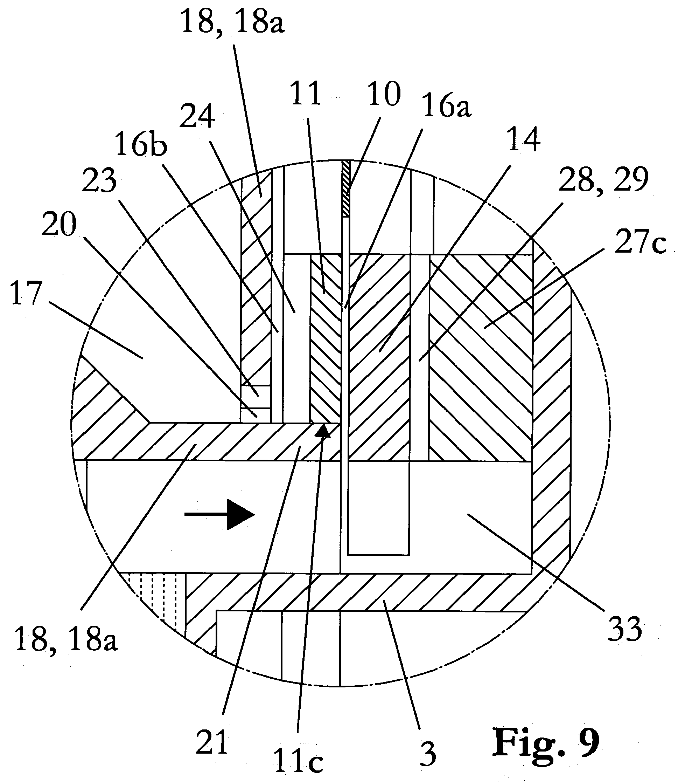

WO 2010/045671 (Helmut Buchberger) beschreibt eine Inhalatorkomponente für die intermittierende, inhalations- oder zugsynchrone Bildung eines Dampf-Luft-Gemisches oder/und Kondensationsaerosols, bestehend aus (Fig. 9-12 und Fig. 17-18) einem Gehäuse 3, eine im Gehäuse 3 angeordnete Kammer 21, eine Lufteinlaßöffnung 26 für die Zufuhr von Luft aus der Umgebung in die Kammer 21, ein elektrisches Heizelement zur Verdampfung einer Portion eines flüssigen Materials 16, wobei der gebildete Dampf sich in der Kammer 21 mit der durch die Lufteinlaßöffnung 26 zugeführten Luft mischt, und sich das Dampf-Luft-Gemisch oder/und Kondensationsaerosol bildet. Die Inhalatorkomponente umfaßt ferner einen Docht mit einer Kapillarstruktur, welcher Docht mit dem Heizelement einen flächigen Verbund 22 bildet und das Heizelement nach einer Verdampfung von neuem selbsttätig mit dem flüssigen Material 16 versorgt. Der flächige Verbund 22 lagert mit zwei Endabschnitten auf zwei elektrisch leitenden, plattenförmigen Kontakten 23, auf deren Oberfläche das Heizelement gleichzeitig elektrisch kontaktiert ist. Die plattenförmigen Kontakte 23 können alternativ auch durch Leiterplatten oder eine gemeinsame Leiterplatte gebildet werden. Zumindest ein beheizter Abschnitt des flächigen Verbundes 22 ist berührungsfrei in der Kammer 21 angeordnet, und die Kapillarstruktur des Dochts liegt im besagten Abschnitt wenigstens auf einer Seite 24 des flächigen Verbundes weitgehend frei. Der flächige Verbund 22 bzw. dessen Docht ragt mit einem Ende in einen Kapillarspalt 41, welcher seinerseits mit einem das flüssige Material 16 enthaltenden Flüssigkeitsbehälter 4 kapillar gekoppelt bzw. koppelbar ist. Der Flüssigkeitsbehälter 4 weist einen öffenbaren Verschluß 18 auf, welcher vor Gebrauch noch verschlossen ist. Der öffenbare Verschluß 18 kann von einem Benutzer manuell geöffnet werden, worauf das flüssige Material 16 ein Reservoir 45 flutet und den Kapillarspalt 41 benetzt. Der Kapillarspalt 41 zieht das flüssige Material 16 aus dem Flüssigkeitsbehälter 4 bzw. Reservoir 45 und transportiert es zum Verbund 22. Der Kapillarspalt 41 wird im Grunde durch einen der beiden plattenförmigen Kontakte 23 und ein auf diesen flächig aufgesetztes Oberteil 42 gebildet, indem die beiden aneinander grenzenden Bauelemente bzw. deren Oberflächen Begrenzungswände des Kapillarspalts 42 bilden. Des Weiteren ist in den plattenförmigen Kontakt 23 ein Belüftungskanal 52 eingearbeitet, welcher das Reservoir 45 bzw. den Flüssigkeitsbehälter 4 mit der Kammer 21 verbindet. Der Belüftungskanal 52 bewirkt einen Druckausgleich, indem jede Portion flüssigen Materials 16, welche in den Kapillarspalt 41 gelangt, unmittelbar durch eine volumengleiche Portion Luft ersetzt wird.

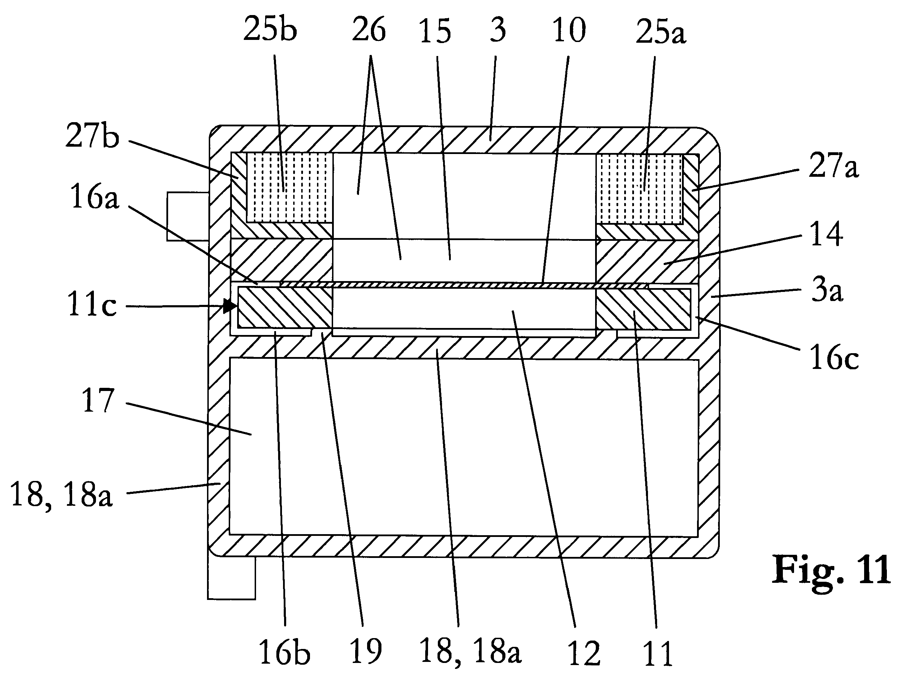

Schließlich ist in das Oberteil 42 ein Pufferspeicher 53 integriert, welcher mit dem Kapillarspalt 41 kommuniziert und selbst aus Kapillaren besteht- siehe Fig. 11 und Fig. 17. Der Pufferspeicher 53 hat die Fähigkeit, flüssiges Material 16 aus dem Kapillarspalt 41 aufzunehmen, zwischenzuspeichern und bei Bedarf wieder an den Kapillarspalt 41 abzugeben. Dadurch kann die Inhalatorkomponente auch in einer umgedrehten Gebrauchsstellung - das Mundstück 5 weist nach unten - betrieben werden, zumindest solange wie flüssiges Material 16 im Pufferspeicher 53 vorrätig ist. Der Pufferspeicher 53 besteht aus parallel zueinander angeordneten Schlitzen 54, welche in das Oberteil 42 eingearbeitet sind. Die Schlitze 54 kommunizieren einerseits über Öffnungen 55 mit dem Kapillarspalt 41 und andererseits über einen Belüftungsspalt 56 mit der Kammer 21. Die Kapillarität der Schlitze 54 bewirkt, daß das flüssige Material 16 aus dem Reservoir 45 über den Kapillarspalt 41 und über die Öffnungen 55 in die Schlitze 54 strömt, wo es zwischengespeichert wird, und vom Kapillarspalt 41 nach Bedarf wieder abgezogen werden kann.

Die Konstruktion des Pufferspeichers 53 erweist sich als ausgesprochen raumfordernd. Die Herstellung der feinen, in das Oberteil 42 eingearbeiten Schlitze 54 samt Öffnungen 55 ist zudem relativ aufwendig. Schließlich ist als Nachteil zu werten, daß die Öffnungen 55 die Kapillarität des Kapillarspalts 41 stören, weil durch die Öffnungen 55 sonst benetzbare Wandabschnitte des Kapillarspalts 41 entfallen. Die Störung der Kapillarität kann im schlimmsten Fall die Versorgung des flächigen Verbundes 22 mit dem flüssigen Material 16 beeinträchtigen.

Der Erfindung liegt die Aufgabe zugrunde, die zuvor aufgezeigten Nachteile der aus dem Stand der Technik bekannten Anordnung zu beheben. Der Erfindung liegt insbesondere die Aufgabe zugrunde, eine Inhalatorkomponente der eingangs geschilderten Art durch konstruktiv einfache Mittel so auszugestalten, daß ausreichende Mengen flüssigen Materials gepuffert werden können, ohne einen wesentlichen zusätzlichen Bauraum zu beanspruchen. Des Weiteren soll die Versorgungssicherheit des Verbundes mit dem flüssigen Material erhöht werden.

Die Aufgabe wird durch die kennzeichnenden Merkmale des Patentanspruches 1 gelöst. Demnach ist vorgesehen, daß sowohl die Vorderseite als auch die Rückseite der Trägerplatte zumindest abschnittsweise Begrenzungswände des Kapillarspalts bilden. Die Versorgung des Verbundes mit dem flüssigen Material erfolgt also nicht bloß auf einer Seite der Trägerplatte, sondern beidseitig. Auf beiden Seiten der Trägerplatte sind Kapillarspalten bzw. Kapillarspalt-Abschnitte vorgesehen, welche von der Trägerplatte begrenzt werden. Hierdurch kann auf einfache und dabei bauraumsparende Weise ein zusätzliches Kapillarspalt-Volumen geschaffen werden, welches gleichzeitig als Puffer wirkt. Ein weiterer günstiger Effekt ist in der Redundanz der Flüssigkeitsversorgung zu sehen: fällt die Versorgung in einem Kapillarspalt-Abschnitt - aus welchem Grund auch immer - aus, so kann der Verbund zumindest noch über den auf der gegenüberliegenden Seite der Trägerplatte liegenden Kapillarspalt-Abschnitt mit dem flüssigen Material versorgt werden.

In einer Weiterbildung der Erfindung ist vorgesehen, daß auch der Trägerplattenrand zumindest abschnittsweise eine Begrenzungswand des Kapillarspalts bildet. Auf diese Weise kann das Puffervolumen weiter vergrößert werden. Besonders günstig ist es, wenn der Kapillarspalt die Trägerplatte zumindest teilweise umschließt. Die Umschließung hat den Effekt, daß die Kapillarspalt-Abschnitte auf der Vorder- und Rückseite der Trägerplatte über den Trägerplattenrand miteinander kommunizieren. Selbst wenn die kapillare Flüssigkeitsströmung an mehreren Stellen des Kapillarspalts unterbrochen wäre, fände sich zumeist ein ein alternativer Pfad zur Umgehung der betroffenen Stellen.

Nach der Erfindung bilden über 50 Prozent der Trägerplatten-Oberfläche Begrenzungswände des Kapillarspalts. Aus dem Gehäuse ragende Abschnitte der Trägerplatte bleiben bei der Berechnung unberücksichtigt. Durch die großflächige Nutzung der Trägerplatten-Oberfläche als Begrenzungswand des Kapillarspalts können die zuvor aufgeführten Effekte betreffend die Bildung eines zusätzlichen Puffervolumens sowie die Erhöhung der Versorgungssicherheit maximiert werden. Ferner kann die Versorgungskapazität, das ist die durch den Kapillarspalt pro Zeiteinheit maximal übertragbare Menge flüssigen Materials, vergrößert werden.

Besonders vorteilhaft ist es, wenn der Kapillarspalt zumindest abschnittsweise durch die Trägerplatte und eine benachbarte Wand des Gehäuses gebildet wird. Der Kapillarspalt wird in diesem Fall zumindest abschnittsweise alleine durch vorhandene Komponenten gebildet. Ohnehin vorhandene Wandabschnitte des Gehäuses werden als Begrenzungswände des Kapillarspalts genutzt. Es wird kein zusätzlicher Bauraum beansprucht.

Eine bevorzugte Ausführungsform der Erfindung betrifft eine Inhalatorkomponente mit einem das flüssige Material enthaltenden Flüssigkeitsbehälter, von welchem der Kapillarspalt das flüssige Material bezieht, und es ist vorgesehen, daß der Kapillarspalt zumindest abschnittsweise durch die Trägerplatte und eine benachbarte Wand des Flüssigkeitsbehälters gebildet wird. Der Flüssigkeitsbehälter kann entweder ein selbständiges Bauteil bilden, oder ein Teil des Gehäuses sein. Im letzteren Fall wird der Flüssigkeitsbehälter durch Wände des Gehäuses gebildet. Besonders günstige Verhältnisse ergeben sich, wenn der Kapillarspalt über eine Versorgungsöffnung in der Wand des Flüssigkeitsbehälters mit dem flüssigen Material im Flüssigkeitsbehälter kommuniziert, indem die Wand des Flüssigkeitsbehälters am Rand der Versorgungsöffnung einen Fortsatz bildet, an welchen Fortsatz die Trägerplatte mit ihrem Rand stößt. Alternativ kann auch vorgesehen sein, daß die Trägerplatte mit ihrer Vorder- oder Rückseite auf dem Fortsatz aufliegt. Demnach sind für die Anbindung des Kapillarspalts an den Flüssigkeitsbehälter keine zusätzlichen Hilfsmittel erforderlich. Durch den Fortsatz wird ein Wandabschnitt der Versorgungsöffnung nach außen verlängert. Setzt man voraus, daß die beteiligten Oberflächen vom flüssigen Material gut benetzbar sind, dann ergibt sich der Effekt, daß eine kleine Menge flüssigen Materials durch die am verlängerten Wandabschnitt wirkenden Adhäsionskräfte aus der Versorgungsöffnung herausgezogen wird. Der Effekt reicht aus, daß das flüssige Material auch die mit ihrem Rand an den Fortsatz stoßende bzw. mit ihrer Vorder- oder Rückseite auf dem Fortsatz aufliegende Trägerplatte erreicht und benetzt. Der Kapillarspalt ist damit mit dem flüssigen Material im Flüssigkeitsbehälter gekoppelt und kann sich, getrieben durch die in ihm wirkenden Kapillarkräfte, mit dem flüssigen Material füllen.

Die Erfindung betrifft außerdem einen Inhalator, umfassend eine erfindungsgemäße Inhalatorkomponente wie zuvor beschrieben. Die Inhalatorkomponente kann also auch nur ein Teil, insbesondere ein auswechselbares Teil eines Inhalators sein.

Die Erfindung wird anhand eines Ausführungsbeispiels gemäß den Zeichnungen näher erläutert. Es zeigen:
Fig. 1 einen erfindungsgemäßen Inhalator in verschiedenen Ansichten;
Fig. 2 den Inhalator nach Fig. 1 mit einem wiederverwendbaren Inhalatorteil und einer auswechselbaren Inhalatorkomponente im entkoppelten Zustand;
Fig. 3a und Fig 3b die auswechselbare Inhalatorkomponente in verschiedenen Ansichten;
Fig. 4a, Fig. 4b, Fig. 4c, Fig. 4d, Fig. 4e, Fig. 4f, Fig. 4g Schnittansichten der auswechselbaren Inhalatorkomponente längs der Linie A-A in Fig. 3b in verschiedenen Montagezuständen;
Fig. 5 das Detail a aus Fig. 4a in einer vergrößerten Darstellung;
Fig. 6 das Detail b aus Fig. 4b in einer vergrößerten Darstellung;
Fig. 7 eine als Multilayer-Leiterplatte ausgeführte Trägerplatte;
Fig. 8 eine Schnittansicht der auswechselbaren Inhalatorkomponente längs der Linie B-B in Fig. 3b;
Fig. 9 das Detail c aus Fig. 8 in einer vergrößerten Darstellung;
Fig. 10 einen Querschnitt der auswechselbaren Inhalatorkomponente in Höhe der Versorgungsöffnung;
Fig. 11 einen Querschnitt der auswechselbaren Inhalatorkomponente in Höhe der Verbunde;
Fig. 12 eine alternative Ausgestaltung des Details c (vgl. Fig. 9).

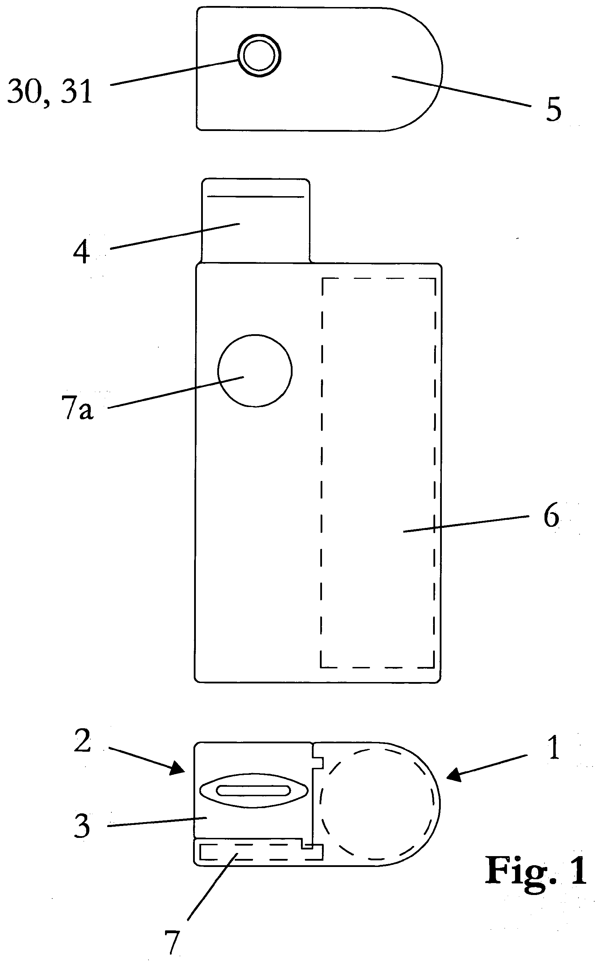

Fig. 1 zeigt einen erfindungsgemäßen Inhalator, dessen Form und Größe derart gestaltet sind, daß der Inhalator von Benutzern einfach und bequem gehandhabt werden kann. Volumenmäßig ist der Inhalator nur etwa halb so groß wie eine Zigarettenschachtel. Der beispielhaft dargestellte Inhalator besteht grundsätzlich aus zwei Teilen, nämlich aus einem Inhalatorteil 1 und einer Inhalatorkomponente 2.

Die Inhalatorkomponente 2 besteht aus einem Gehäuse 3, welches an einer Stirnseite ein tabakpfeifenartiges Mundstück 4 ausbildet. Das Gehäuse 3 ist vorzugsweise aus Kunststoff gefertigt. Die Inhalatorkomponente 2 beinhaltet ein flüssiges Material, welches innerhalb des Gehäuses 3 elektrisch verdampft wird und in ein inhalierbares Dampf-Luft-Gemisch oder/und Kondensationsaerosol übergeführt wird. Das gebildete Dampf-Luft-Gemisch oder/und Kondensationsaerosol wird dem Benutzer über das Mundstück 4 dargeboten. Als flüssiges Material kommen grundsätzlich alle Stoffe und Zubereitungen in Betracht, welche unter atmosphärischen Bedingungen weitgehend rückstandsfrei verdampfen. Diese Bedingung ist auch schon erfüllt, wenn der jeweilige Stoff oder die jeweilige Zubereitung verdünnt, beispielsweise in Wasser oder/ und Ethanol gelöst vorliegt und die Lösung weitgehend rückstandsfrei verdampft. Durch eine hinreichend hohe Verdünnung in einem leicht flüchtigen Lösungsmittel wie Wasser oder/und Ethanol können auch sonst schwer verdampfbare Stoffe die zuvor genannte Bedingung erfüllen, und eine thermische Zersetzung des flüssigen Materials vermieden oder deutlich verringert werden. Die durch Kondensation erzeugten Aerosolteilchen weisen in der Regel einen massemedianen aerodynamischen Durchmesser (MMAD) kleiner als 2µm auf und erreichen dadurch auch die Alveolen. Der erfindungsgemäße Inhalator eignet sich insbesondere für die Verabreichung von systemisch wirkenden Stoffen - insbesondere solchen Wirkstoffen, welche ihre Hauptwirkung im zentralen Nervensystem entfalten. Als Beispiel sei Nikotin erwähnt, dessen Siedepunkt bei 246°C liegt. Die nikotinhaltigen Aerosolpartikel werden vorwiegend in den Bronchien und Alveolen niedergeschlagen, wo der Wirkstoff blitzartig in den Blutkreislauf übergeht. Wenige Sekunden später erreicht das Nikotin in gebündelter Konzentration das Gehirn und kann dort die bekannten Wirkungen entfalten.

Das Inhalatorteil 1 besteht aus einem Hauptgehäuse 5, welches vorzugsweise wieder aus Kunststoff gefertigt ist. Das Hauptgehäuse 5 beinhaltet zumindest eine Batterie 6 und einen elektrischen Schaltkreis 7 (in Fig. 1 strichliert dargestellt) samt Schalter 7a. Die Batterie 6 und der elektrische Schaltkreis 7 stellen die für die Verdampfung des flüssigen Materials erforderliche elektrische Energie bereit. Die Batterie 6 besteht vorzugsweise aus einem wiederaufladbaren Akkumulator, beispielsweise vom Typ CGR18650K des Herstellers Panasonic, www.industrial.panasonic.com. Hierbei handelt es sich um eine zylindrische Lithium-Ionen-Zelle der Baugröße 18650 mit einer Speicherkapazität von 1650mAh und einer Strom-Belastbarkeit von bis zu 30A. Vergleichbare Zellen werden auch von anderen Herstellern, u.a. Sony, Samsung, LG Chem, in großen Stückzahlen gefertigt.

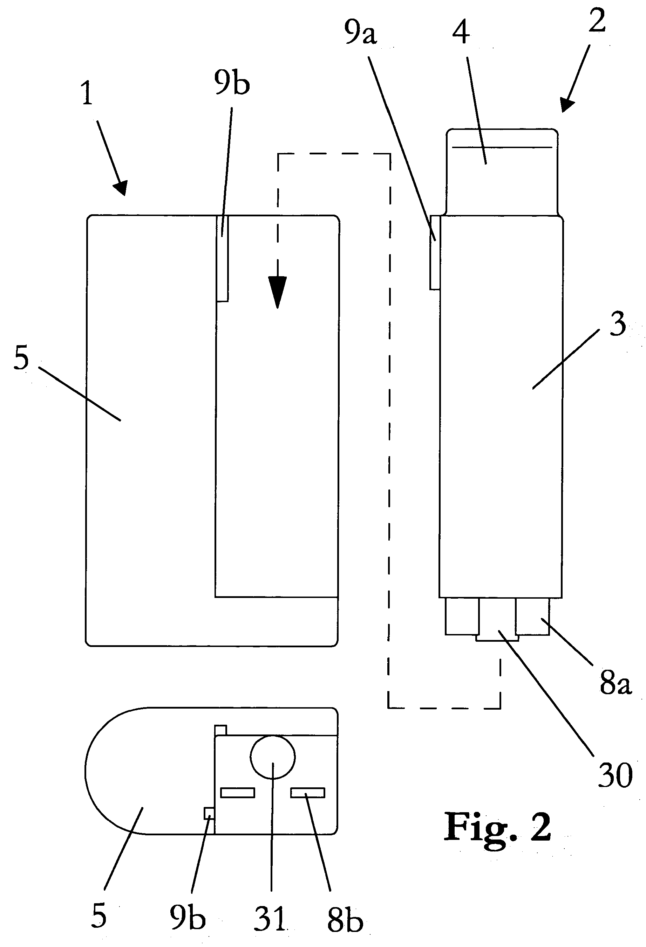

Wie die Fig. 2 zeigt, sind das Inhalatorteil 1 und die Inhalatorkomponente 2 im konkreten Ausführungsbeispiel voneinander lösbar ausgeführt. Diese Anordnung macht das Inhalatorteil 1 wiederverwendbar, was grundsätzlich sinnvoll ist, wenn man in Betracht zieht, daß erstens das Inhalatorteil 1 mit dem flüssigen Material nicht in Berührung kommt, also nicht mit dem flüssigen Material kontaminiert wird, und zweitens Komponenten beinhaltet, welche langlebiger sind als die Bestandteile der Inhalatorkomponente 2. Die Inhalatorkomponente 2 wird, nachdem das flüssige Material aufgebraucht ist, vom Benutzer als Ganzes sachgerecht entsorgt, und durch eine neue Inhalatorkomponente 2 ersetzt. Die Inhalatorkomponente 2 stellt insofern einen auswechselbaren Einwegartikel dar. Eine sachgerechte Entsorgung ist vor allem dann angezeigt, wenn das flüssige Material Arzneimittel oder Gifte wie Nikotin enthält. Grundsätzlich wäre es natürlich auch denkbar, das Inhalatorteil 1 und die Inhalatorkomponente 2 einteilig, also voneinander untrennbar auszuführen. Diese Ausführungsform dürfte jedoch unwirtschaftlicher sein, weil in diesem Fall alle Teile und Komponenten des Inhalators, also der Inhalator als Ganzes einen Einwegartikel zur einmaligen Benutzung bildet. Selbstverständlich schließt die gegenständliche Erfindung auch diese Ausführungsform mit ein, wobei in diesem Fall der ganze Inhalator als Inhalatorkomponente aufzufassen ist.

Die mechanische Kopplung zwischen der auswechselbaren Inhalatorkomponente 2 und dem wiederverwendbaren Inhalatorteil 1 erfolgt über Steckzungen 8a und durch das Gehäuse 3 gebildete Führungsnasen 9a, welche in korrespondierende, durch das Hauptgehäuse 5 des wiederverwendbaren Inhalatorteils 1 gebildete Steckbuchsen 8b und Führungsnuten 9b eingreifen. Die Steckzungen 8a und Steckbuchsen 8b dienen gleichzeitig zur Einleitung der elektrischen Energie in die auswechselbare Inhalatorkomponente 2 zur Verdampfung des flüssigen Materials, wie nachfolgend noch detaillierter gezeigt wird.

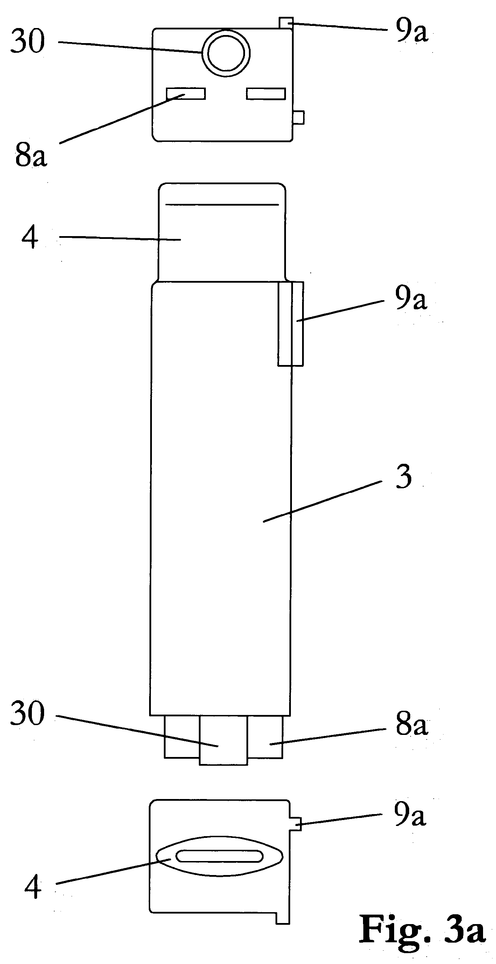

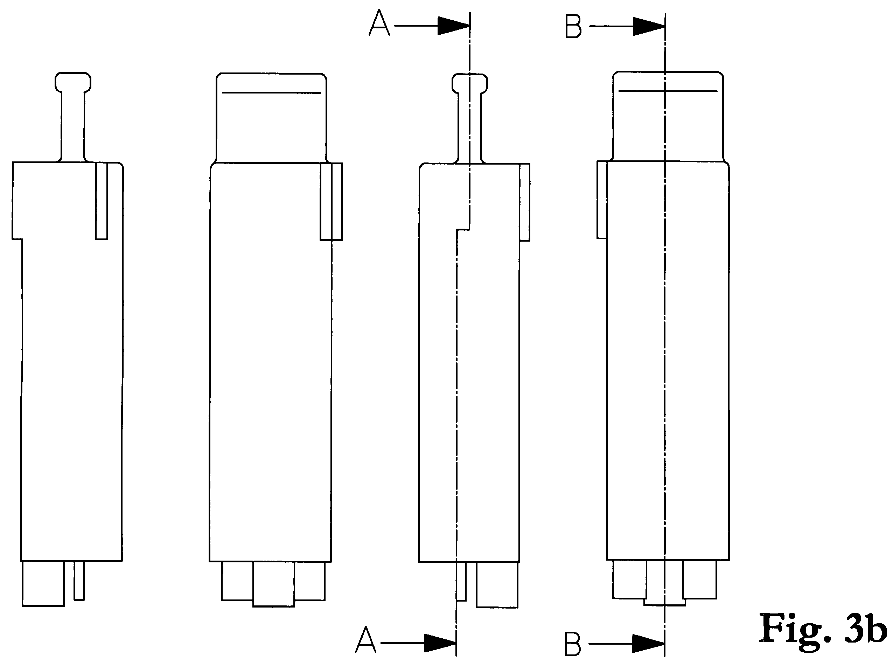

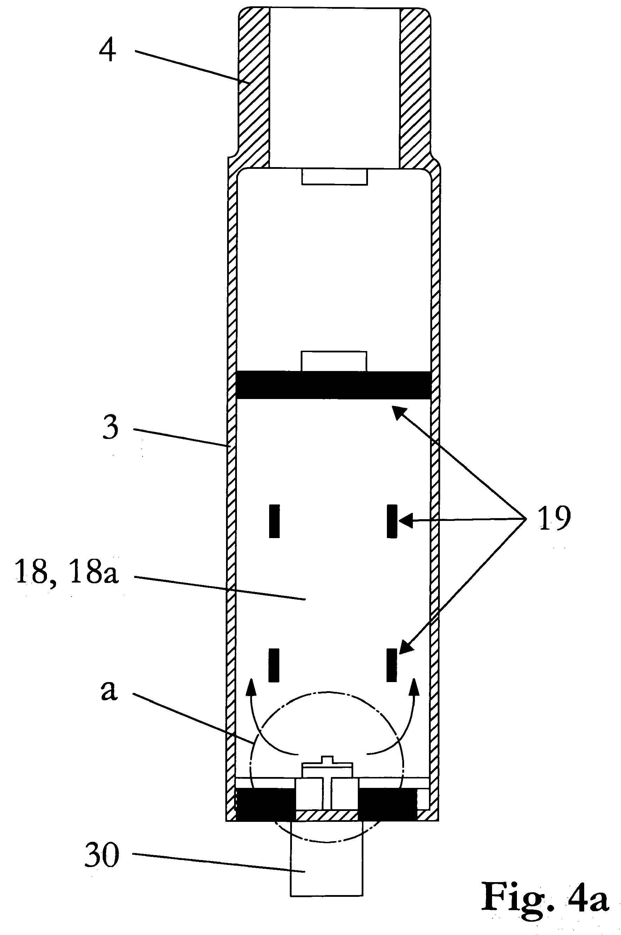

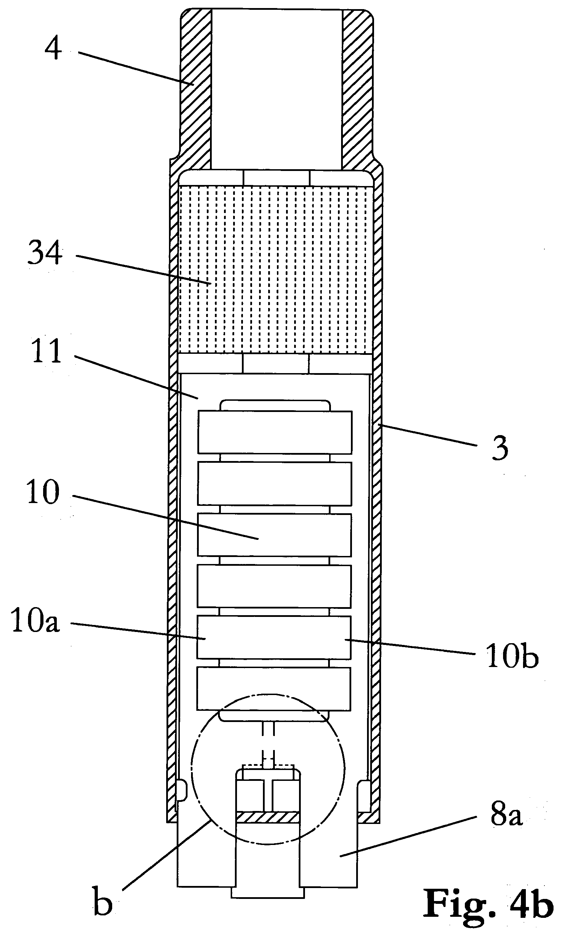

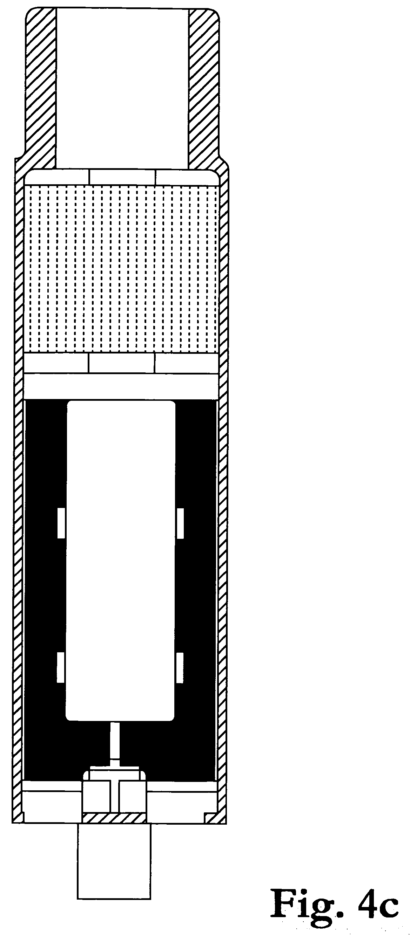

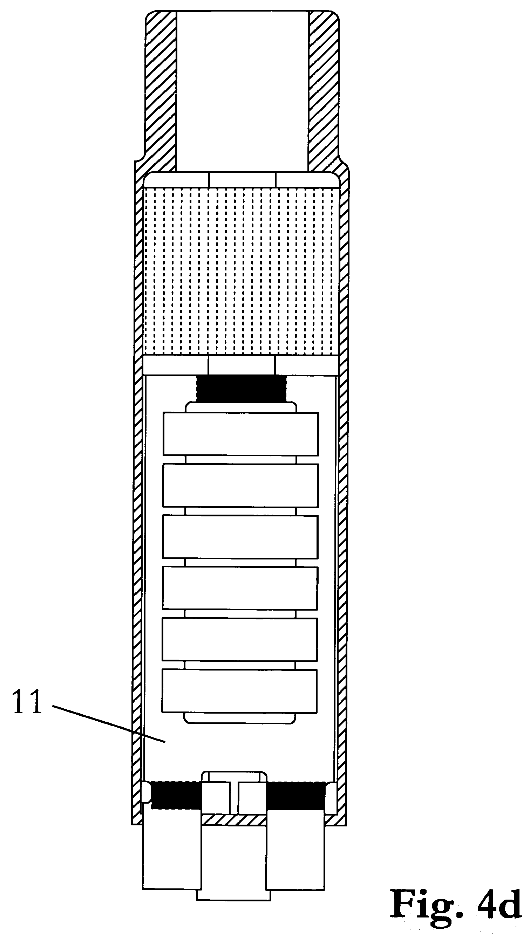

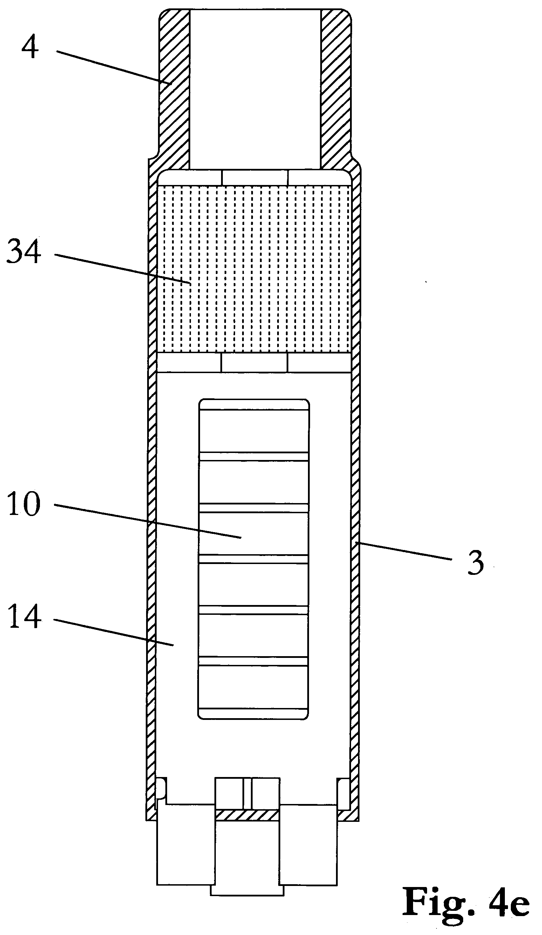

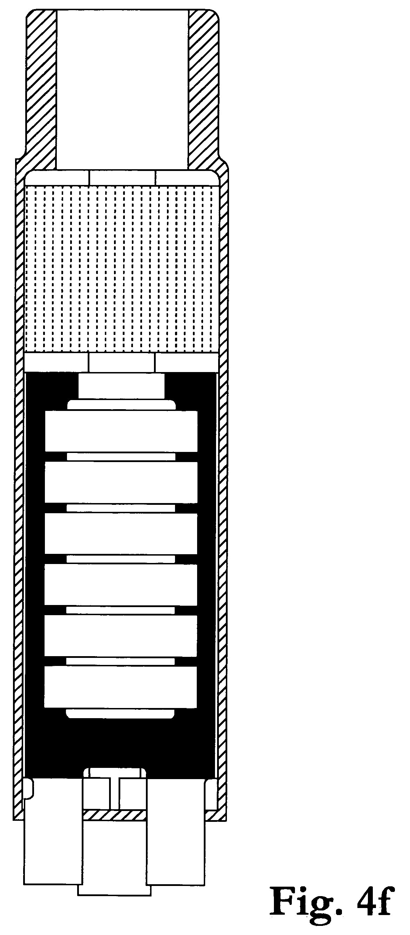

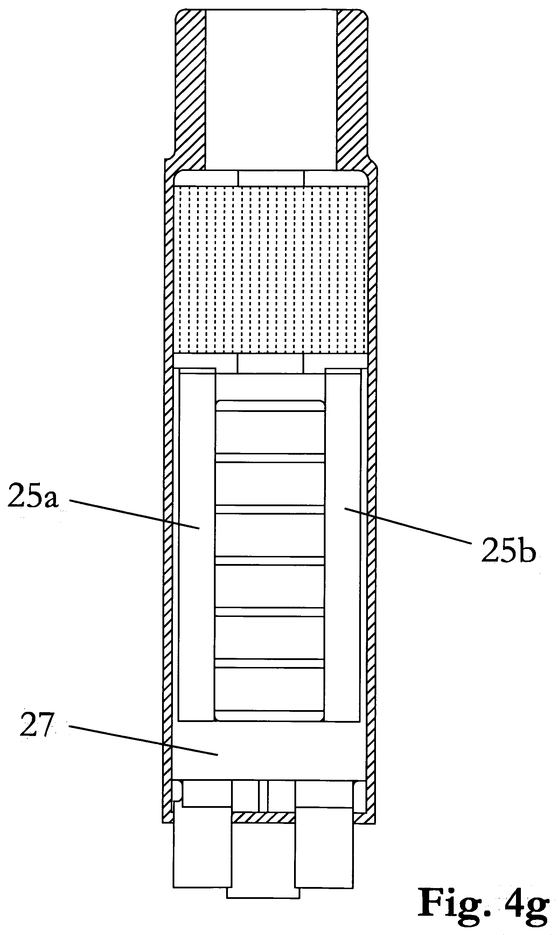

Fig. 3a und Fig. 3b zeigen verschiedene Ansichten der auswechselbaren Inhalatorkomponente 2. Die Fig. 4-11 geben weiteren Aufschluss über den inneren Aufbau der Inhalatorkomponente 2. Demnach weist das Gehäuse 3 der Inhalatorkomponente 2 im Wesentlichen eine quaderförmige Gestalt auf. Im Inneren des quaderförmigen Gehäuses 3 befinden sich die für die Bildung des Dampf-Luft-Gemisches oder/und Kondensationsaerosols wesentlichen Komponenten. Zu diesen zählen insbesondere die Verbunde 10, welche die Verdampfung des flüssigen Materials bewirken. Im konkreten Ausführungsbeispiel sind sechs Verbunde 10 nebeneinander angeordnet, und die Verbunde haben eine flächige Gestalt. Die flächigen Verbunde 10 bestehen jeweils aus einem Docht und einem elektrischen Heizelement, welche miteinander flächig verbunden oder flächig ineinander integriert sind. Die flächigen Verbunde 10 können beispielsweise durch eine Metallfolie und darauf aufgesinterte Metallgewebelagen gebildet werden. Statt des Metallgewebes können auch offenporige Metallschäume Verwendung finden. Die offenporige Kapillarstruktur der auf die Metallfolie aufgesinterten Gewebelagen bzw. des Metallschaums bildet den Docht, und der elektrische Widerstand des Metalls bildet das Heizelement. Geeignete metallische Widerstandsmaterialien sind beispielsweise Edelstähle wie AISI 304 oder AISI 316 sowie Heizleiterlegierungen, insbesondere NiCr-Legierungen. Die Herstellung solcher flächigen Verbunde 10 zählt zum Stand der Technik und wird beispielsweise in der bereits zitierten WO 2010/045671 (Helmut Buchberger) im Detail offenbart. Angemerkt sei, daß die flächigen Verbunde 10 nicht zwingend eben ausgebildet sein müssen, sondern auch eine räumliche Krümmung aufweisen können.

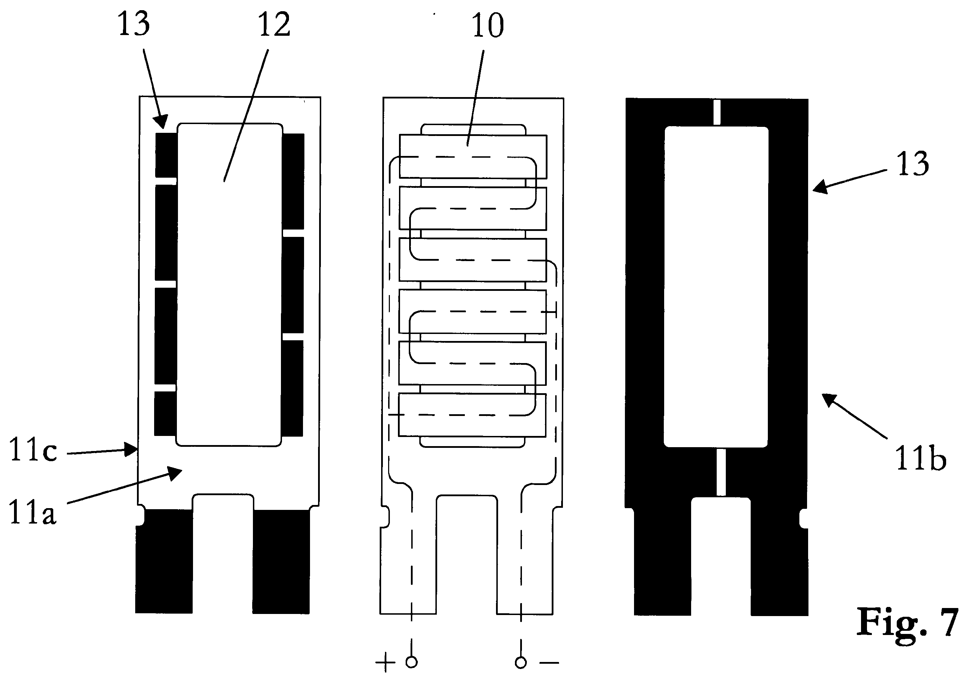

Wie Fig. 4b und Fig. 7 am besten zeigen, lagern die flächigen Verbunde 10 mit zwei Endabschnitten 10a, 10b auf einer Trägerplatte 11. Die Trägerplatte 11 weist eine große Ausnehmung 12 auf, welche von den Verbunden 10 berührungsfrei überspannt wird. Die Trägerplatte 11 ist im konkreten Ausführungsbeispiel als Leiterplatte, insbesondere als Multilayer-Leiterplatte ausgeführt. Als Material für die Leiterplatte 11 eignen sich grundsätzlich alle bekannten Leiterplatten-Werkstoffe, insbesondere die Werkstofftypen FR1 bis FR5. Die flächigen Verbunde 10 sind im Bereich der Endabschnitte 10a, 10b auf Leiterbahnen 13 der Leiterplatte 11 elektrisch kontaktiert. In Fig. 7 sind die Leiterbahnen 13 als schwarze Flächen dargestellt. Im Fall der zuvor beschriebenen Metallfolien-Verbunde erfolgt die elektrische Kontaktierung vorzugsweise durch eine folienseitige Lötung, gegebenenfalls nach Vorbehandlung mit einem geeigneten Flussmittel. Edelstähle der Werkstoff-Qualitäten AISI 304 und AISI 316 können beispielsweise mit einem Lötkonzentrat mit der Handelbezeichnung "5050S-Nirosta" der Firma Stannol GmbH, www.stannol.de, problemlos gelötet werden. Die elektrische Kontaktierung kann alternativ aus einer Klebeverbindung mittels eines elektrisch leitenden Klebstoffes, zum Beispiel mittels eines silberhaltigen Klebers auf Epoxidbasis, bestehen. Die Bestückung der Leiterplatte 11 mit den flächigen Verbunden 10 sowie deren Kontaktierung erfolgen vollautomatisch, wobei Verfahren der Leiterplattenindustrie angewandt werden können, welche Verfahren sich im Übrigen auch für eine Massenfertigung eignen.

Die Leiterplatte 11 ragt aus dem Gehäuse 3 in Form der bereits früher erwähnten Steckzungen 8a heraus. Die beiden Steckzungen 8a dienen zur Einleitung der elektrischen Energie in die Inhalatorkomponente 2. Die elektrische Energie wird den Verbunden 10 über die Leiterbahnen 13 zugeführt. Nach Fig. 7 sind die Leiterbahnen 13 sowohl auf der Vorderseite 11a als auch auf der Rückseite 11b der Leiterplatte 11 angeordnet, wobei die Vorderseite 11a die Bestückungsseite ist - das ist jene Seite, auf welcher die Verbunde 10 kontaktiert sind. Weitere Leiterbahnen können optional auch noch in Zwischenlagen angeordnet werden. Die einzelnen Leiterbahn-Lagen sind nach dem Stand der Technik mittels sogenannter Durchkontaktierungen zweckmäßig miteinander verbunden. In Fig. 7 ist ferner der Stromfluß dargestellt. Demnach sind im konkreten Beispiel jeweils drei Verbunde 10 miteinander in Reihe geschaltet. Dadurch kann auf den resultierenden Heizwiderstand und damit auf die Heizleistung und Verdampfungrate in gewissen Grenzen Einfluß genommen werden. Es kann auch vorgesehen sein, daß die elektrischen Einzelwiderstände der sechs Verbunde 10 unterschiedlich groß sind, indem beispielsweise die Dicke der Metallfolie entsprechend variiert wird. Durch diese Maßnahme kann der Verdampfungsprozeß ähnlich wie in einer Zigarette auch vom Ort abhängig gemacht werden.

Auf die Vorderseite 11a der Leiterplatte 11 ist ein im Wesentlichen plattenförmiges, vorzugsweise aus Kunststoff bestehendes Oberteil 14 aufgesetzt (siehe Fig. 4c und Fig. 8-10). Das Oberteil 14 weist eine Aussparung 15 auf, welche hinsichtlich ihrer Größe und Anordnung mit der Ausnehmung 12 in der Leiterplatte 11 korreliert. Im einfachsten Fall lagert das Oberteil 14 direkt auf den Endabschnitten 10a, 10b der flächigen Verbunde 10. Hierdurch bildet das Oberteil 14 zusammen mit der Leiterplatte 11 einen ersten Kapillarspalt-Abschnitt 16a, dessen lichte Weite bzw. Spaltbreite im Wesentlichen der Dicke der flächigen Verbunde 10 entspricht (siehe Fig. 9 und Fig. 11). Die Spaltbreite beträgt typischerweise 0,2mm. In Fig. 4f ist die flächige Ausdehnung des ersten Kapillarspalt-Abschnitts 16a als schwarze Fläche dargestellt. Das Oberteil 14 ist auf der Leiterplatte 11 durch eine Klebeverbindung befestigt. Die Klebestellen sind in Fig. 4d als schwarze Flächen dargestellt. Die Leiterplatte 11 und das Oberteil 14 werden vorzugsweise außerhalb des Gehäuses 3 gefügt, stellen also eine vormontierte Baueinheit dar.

Die Leiterplatte 11 lagert mit ihrer Rückseite 11b zumindest abschnittsweise auf einem das flüssige Material 17 enthaltenden, quaderförmigen Flüssigkeitsbehälter 18 (siehe Fig. 4a/4b, Fig. 8-9 und Fig. 11). Der Flüssigkeitsbehälter 18 bzw. dessen Wände 18a werden durch das Gehäuse 3 gebildet. Die Leiterplatte 11 lagert jedoch nicht unmittelbar auf der Flüssigkeitsbehälterwand 18a, sondern auf Abstandshaltern 19. Die Abstandshalter 19 werden teils durch die Flüssigkeitsbehälterwand 18a und teils durch sonstige Gehäuseabschnitte gebildet; sie sind in Fig. 4a als schwarze Flächen dargestellt. Auf diese Weise wird ein zweiter Kapillarspalt-Abschnitt 16b gebildet. Die Rückseite 11b der Leiterplatte 11 und die benachbarte Flüssigkeitsbehälterwand 18a bilden die Begrenzungswände dieses zweiten Kapillarspalt-Abschnitts 16b. In Fig. 4c ist die flächige Ausdehnung des zweiten Kapillarspalt-Abschnitts 16b als schwarze Fläche dargestellt. Die Spaltbreite wird durch die Höhe der Abstandshalter 19 festgelegt und beträgt typischerweise 0,3mm. Die Leiterplatte 11 ist vorzugsweise mittels einer Klebeverbindung auf den Abstandshaltern 19 befestigt. Die Befüllung des Flüssigkeitsbehälters 18 mit dem flüssigen Material 17 erfolgt werkseitig am Ende des Fertigungsprozesses vorzugsweise über ein kleines Loch in der Behälterwand 18a (nicht dargestellt) vollautomatisch mittels einer Kanüle und einer Dosiereinheit. Das Loch wird nach der Befüllung verschlossen, zum Beispiel zugeschmolzen, und die ganze Inhalatorkomponente 2 luftdicht verpackt.

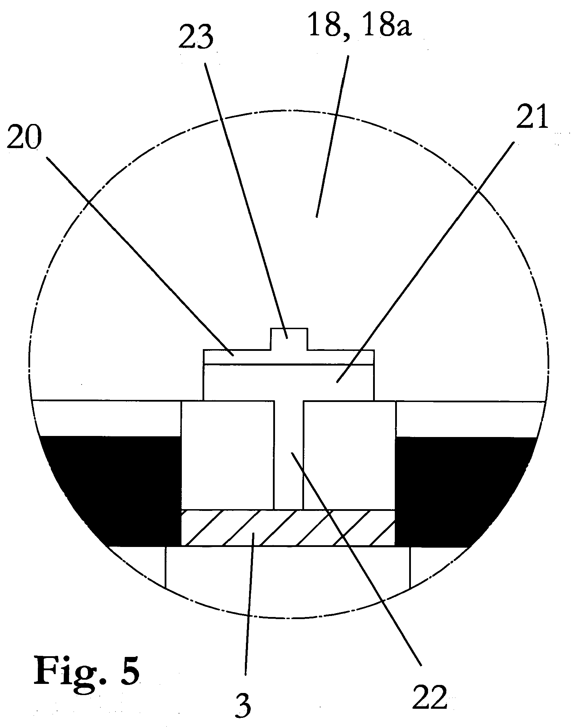

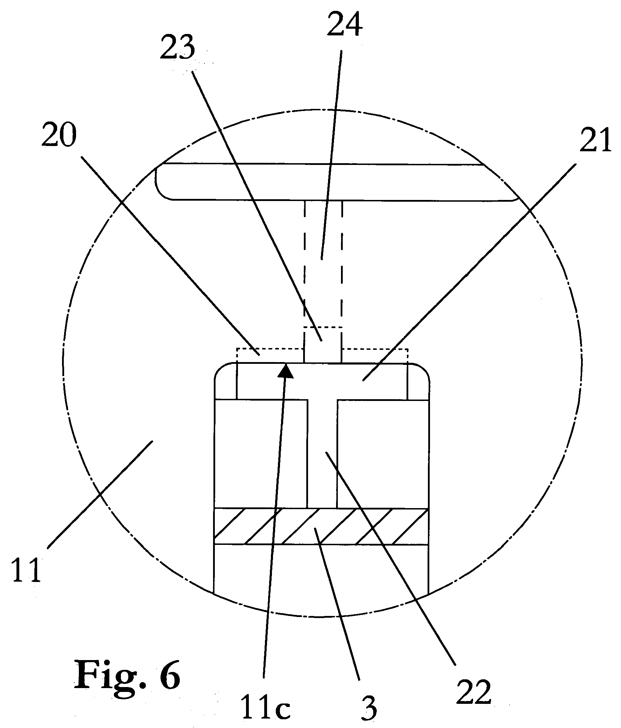

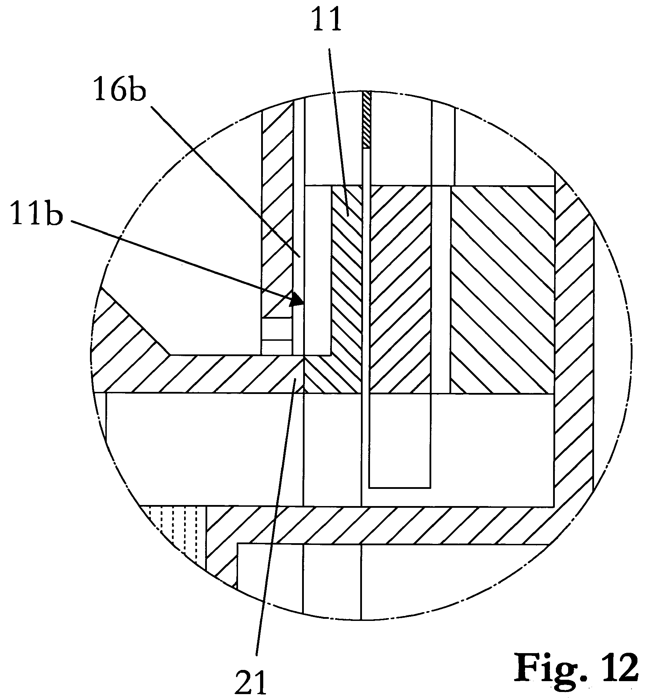

Der Flüssigkeitsbehälter 18 weist an seinem unteren Ende eine schlitzförmige Versorgungsöffnung 20 auf (siehe Fig. 5-6, Fig. 9-10). Der zweite Kapillarspalt-Abschnitt 16b bezieht alles flüssige Material 17 über diese Versorgungsöffnung 20. Die kapillare Kopplung erfolgt über einen durch die Flüssigkeitsbehälterwand 18a gebildeten Fortsatz 21. Durch den Fortsatz 21 wird ein Wandabschnitt der Versorgungsöffnung 20 nach außen verlängert (siehe Fig. 9). Die am verlängerten Wandabschnitt wirkenden Adhäsionskräfte haben zur Folge, daß eine kleine Menge flüssigen Materials 17 aus der Versorgungsöffnung 20 hervortritt. Der Effekt reicht aus, daß das flüssige Material 17 auch die Leiterplatte 11 erreicht, welche mit ihrem Rand 11c an den Fortsatz 21 stößt (siehe Fig. 6 und Fig. 9). In einer alternativen Ausgestaltung liegt die Leiterplatte 11 mit ihrer Rückseite 11b auf dem Fortsatz 21 auf (siehe Fig. 12). Sobald das flüssige Material 17 die Rückseite 11b der Leiterplatte 11 benetzt, kann der zweite Kapillarspalt-Abschnitt 16b seine Saugwirkung entfalten und flüssiges Material 17 aufnehmen. Zur Aussteifung stützt sich der Fortsatz 21 über einen Steg 22 am Gehäuse 3 ab.

Die schlitzförmige Versorgungsöffnung 20 weist etwa mittig eine Aufweitung auf. Die Aufweitung bildet eine Belüftungsöffnung 23. Die Belüftungsöffnung 23 kommuniziert mit einer auf der Leiterplatten-Rückseite 11b in die Leiterplatte 11 eingebrachten Belüftungsnut 24, welche ihrerseits über die Ausnehmung 12 mit einem unter Atmosphärendruck stehenden Innenraum kommuniziert. Die Belüftungsöffnung 23 und die Belüftungsnut 24 bewirken einen Druckausgleich, indem jede Portion flüssigen Materials 17, welche vom zweiten Kapillarspalt-Abschnitt 16b aufgenommen wird, unmittelbar durch eine volumengleiche Portion Luft ersetzt wird.

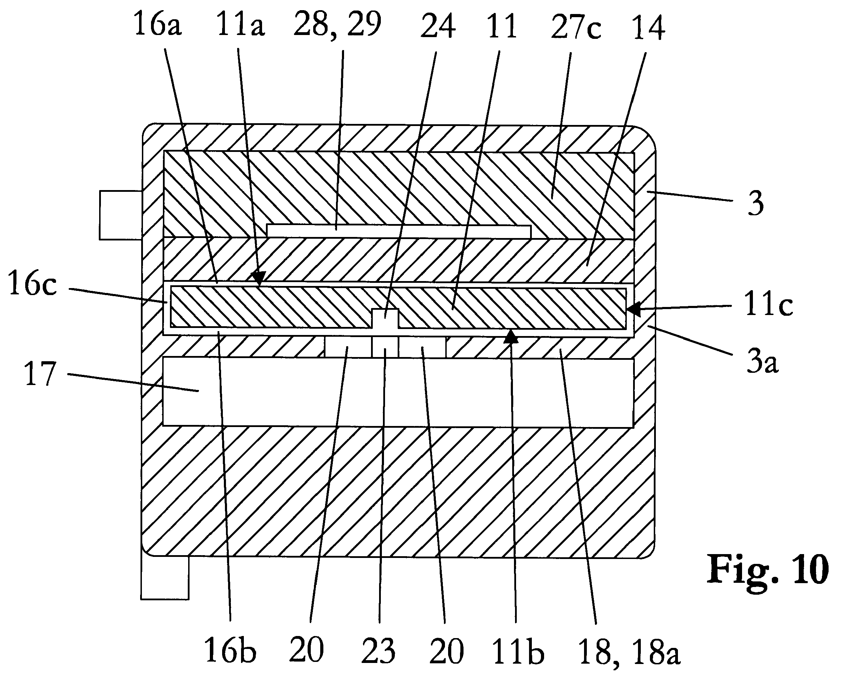

Wie Fig. 10 und 11 am besten zeigen, sind der erste Kapillarspalt-Abschnitt 16a und der zweite Kapillarspalt-Abschnitt 16b über einen dritten Kapillarspalt-Abschnitt 16c miteinander verbunden. Der dritte Kapillarspalt-Abschnitt 16c wird durch den Leiterplattenrand 11c und eine benachbarte Gehäusewand 3a gebildet. Zur exakten Festlegung des dritten Kapillarspalt-Abschnitts 16c dient das mit der Leiterplatte 11 verbundene plattenförmige Oberteil 14, welches an die Gehäusewand 3a angrenzt und den Leiterplattenrand 11c um ein genau definiertes Maß überragt. Das Maß entspricht der Spaltbreite des dritten Kapillarspalt-Abschnitts 16c und beträgt typischerweise 0,3mm. Die Leiterplatte 11 und das plattenförmige Oberteil 14, welche, wie bereits erwähnt wurde, eine vormontierte Baueinheit bilden, müssen also exakt gefügt werden.

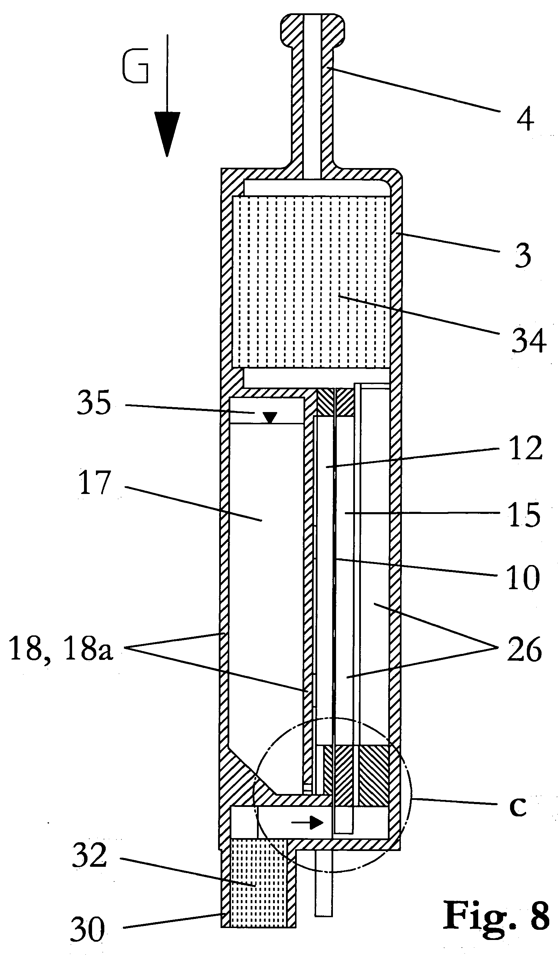

Die drei Kapillarspalt-Abschnitte 16a, 16b, 16c bilden zusammen den Kapillarspalt 16. Der Kapillarspalt 16 besteht also aus einem ausgedehnten, zusammenhängenden Kapillarspalt-System, welches die Leiterplatte 11 teilweise umschließt. Läßt man die aus dem Gehäuse 3 ragenden Abschnitte der Leiterplatte 11, also die Steckzungen 8a, unberücksichtigt, dann bilden im konkreten Ausführungsbeispiel deutlich mehr als 50% der Leiterplatten-Oberfläche Begrenzungswände des Kapillarspalts 16. Die daraus resultierenden vorteilhaften Effekte betreffend die Pufferung des flüssigen Materials 17, sowie betreffend die Versorgungssicherheit und Versorgungskapazität wurden bereits früher dargestellt. Eine Grundvoraussetzung für die Erzielung dieser günstigen Effekte ist, daß das flüssige Material 17 sämtliche beaufschlagten Oberflächen gut benetzt. Um dies sicherzustellen, sind die betroffenen Bauteile - das sind der Flüssigkeitsbehälter 18, die Leiterplatte 11 samt Verbunden 10, das Oberteil 14 und zumindest Teile des Gehäuses 3 - noch vor der Montage in einem geeigneten Prozeß zu hydrophilieren. Geeignete Prozesse sind das Hydrophilieren im Sauerstoff-Plasma sowie das Hydrophilieren mittels Plasmapolymerisation. Beide Prozesse werden beispielsweise von der Firma Diener electronic GmbH u. Co. KG, www.plasma.de, im Rahmen von Lohnaufträgen angeboten. Die genannte Firma ist außerdem in der Lage, entsprechende, auch für eine Massenfertigung taugliche Anlagen kundenspezifisch zu planen und zu errichten. Bevor auf die Funktionsweise des erfindungsgemäßen Inhalators näher eingegangen wird, sollen im Folgenden noch weitere Bestandteile der Inhalatorkomponente 2 beschrieben werden. Auch wenn diese Bestandteile nicht unmittelbar erfindungsrelevant sein mögen, so trägt die Beschreibung derselben doch dazu bei, die Funktion der erfindungsgemäßen Inhalatorkomponente im Ganzen noch besser zu verstehen, und die Ausführbarkeit der Erfindung noch sicherer zu gewährleisten: zwischen dem Oberteil 14 und dem Gehäuse 3 sind zwei offenporige, saugfähige Schwämme 25a, 25b (siehe Fig. 4g und Fig. 11) angeordnet. Der Raum zwischen den Schwämmen bildet zusammen mit der Aussparung 15 eine Kammer 26 (vgl. auch Fig. 8), in welcher die eigentliche Bildung des Dampf-Luft-Gemisches oder/und Kondensationsaerosols stattfindet. Die Schwämme 25a, 25b nehmen in ihren Poren aus der Dampfphase gebildete Kondensatablagerungen auf und verhindern, daß sich in der Inhalatorkomponente 2 frei bewegliche Kondensatansammlungen bilden, welche die Funktion der Inhalatorkomponente beeinträchtigen könnten. Solche Kondensatansammlungen können auch aus hygienischer Sicht ein Problem darstellen, insbesondere wenn sie über das Mundstück 4 in die Mundhöhle eines Benutzers gelangen. Die Schwämme 25a, 25b bestehen vorzugsweise aus einem feinporigen Faserverbund. Die Firma Filtrona Fibertec GmbH, www.filtronafibertec.com, ist auf die Herstellung solcher Faserverbunde spezialisiert, wobei sowohl mittels Triacetin gebundene Celluloseacetat-Fasern als auch thermisch gebundene Polyolefin- und Polyesterfasern verarbeitet weden.

Die Schwämme 25a, 25b lagern auf von einem U-förmigen Träger 27 gebildeten Winkelprofilen 27a, 27b (siehe Fig. 4g und Fig. 11). Der Träger 27 ist mit dem Oberteil 14 durch eine Klebeverbindung gefügt. Der Träger 27 samt Winkelprofilen 27a, 27b besteht vorzugsweise aus einem hydrophoben Kunststoff. Das hydrophobe Material wirkt wie eine Flüssigkeitssperre und stellt sicher, daß kein flüssiges Material 17 durch Kapillareffekte zu den Schwämmen 25a, 25b gelangen kann. In den die Winkelprofile 27a, 27b verbindenden Schenkel 27c ist auf der dem Oberteil 14 zugewandten Seite eine Vertiefung 28 eingearbeitet, welche zusammen mit dem Oberteil 14 eine Luftdüse 29 bildet (siehe Fig. 9 und Fig. 10). Die Luftdüse 29 dient, wie später noch näher dargestellt wird, der Einbringung von Umgebungsluft in die Kammer 26. Damit Kondensatablagerungen die Luftdüse 29 nicht blockieren, empfiehlt es sich, die Oberfäche des Oberteils 14 im Bereich der Luftdüse 29 mit einem dünnen hydrophoben Klebeband zu überkleben (nicht dargestellt).

Die Versorgung der Inhalatorkomponente 2 mit Umgebungsluft zur Bildung des Dampf-Luft-Gemisches oder/und Kondensationsaerosols erfolgt über einen durch das Gehäuse 3 gebildeten Ansaugschnorchel 30 (siehe Fig. 3a/3b und Fig. 8). Der Ansaugschnorchel 30 ist auf der dem Mundstück 4 gegenüberliegenden Seite der Inhalatorkomponente 2 angeordnet. Diese Lage schützt am ehesten vor dem Eintritt von Regenwasser. Im gekoppelten Zustand ragt der Ansaugschnorchel 30 der Inhalatorkomponente 2 durch ein durch das Hauptgehäuse 5 des Inhalatorteils 1 gebildetes Loch 31 (siehe Fig. 2). Im Ansaugschnorchel 30 befindet sich eine Strömungsdrossel 32. Die Strömungsdrossel 32 hat den Zweck, einen Strömungswiderstand zu erzeugen, welcher dem einer Zigarette ähnlich ist, so daß der Benutzer während eines Zuges einen ähnlichen Zugwiderstand verspürt wie bei einem Zug an einer Zigarette. Konkret sollte der Strömungswiderstand bei einem Durchfluß von 1,05 L/min im Bereich 8-16mbar liegen und eine möglichst lineare Charakteristik aufweisen. Die Strömungsdrossel 32 ist erforderlich, wenn das gebildete Dampf-Luft-Gemisch oder/und Kondensationsaerosol wie bei einer Zigarette zugeführt werden soll, nämlich als Zug in die Mundhöhle (Zugvolumen: ca. 20-80mL), gegebenenfalls gefolgt von einer Inhalation in die Lunge. Diese Betriebsweise empfiehlt sich vor allem dann, wenn das flüssige Material 17 Nikotin enthält. Die Strömungsdrossel 32 entfällt jedoch, wenn der Inhalator eine direkte Lungeninhalation in einem einzigen Schritt gestatten soll, wie dies bei den meisten medizinischen Inhalatoren der Fall ist. Die Strömungsdrossel 32 besteht vorzugsweise aus einem zigarettenfilterähnlichen Faserverbund, wobei die Dichte des Materials auf die zuvor genannte Durchflußcharakteristik abzustimmen ist. Das Material kann wiederum von der Firma Filtrona Fibertec GmbH, www.filtronafibertec.com, bezogen werden.

Im Folgenden soll die Funktion des Inhalators im Detail beschrieben werden: ein Benutzer koppelt eine neue Inhalatorkomponente 2 mit dem wiederverwendbaren Inhalatorteil 1. Der elektrische Schaltkreis 7 registriert die Kopplung und veranlaßt gegebenenfalls die Ausführung bestimmter vorbereitender Operationen, beispielsweise eines oder mehrerer Verdampfungszyklen mit dem Ziel, die Verbunde 10 mit frischem flüssigen Material 17 zu versorgen oder/und stationäre Bedingungen herzustellen. Sobald diese Operationen abgeschlossen sind, signalisiert der elektrische Schaltkreis 7 zum Beispiel über eine Leuchtdiode die Betriebsbereitschaft des Inhalators. Der Benutzer führt das Mundstück 4 des Inhalators an den Mund und betätigt den Schalter 7a. Gleichzeitig beginnt er am Mundstück 4 zu ziehen. Der hierdurch erzeugte Unterdruck bewirkt, daß Luft aus der Umgebung in den Ansaugschnorchel 30 strömt. Nachdem die Luft die Strömungsdrossel 32 durchsetzt hat, biegt die Strömung im rechten Winkel ab (siehe Pfeile in Fig. 8 und Fig. 9) und mündet in eine Plenumkammer 33, wo sich die Luft sammelt und sodann gleichmäßig der schlitzförmigen Luftdüse 29 zugeführt wird. Die Luftströmung wird in der Luftdüse 29 beschleunigt und tritt mit einer hohen Mündungsgeschwindigkeit in die Kammer 26 ein.

Die Betätigung des Schalters 7a bewirkt, daß der Schaltkreis 7 den Heizstrom einschaltet. Der Heizstrom wird vorzugsweise mittels Leistungs-MOSFET geschaltet, wobei die zugeführte Leistung durch eine Taktung (Duty Cycle) den jeweiligen Erfordernissen angepaßt werden kann. Diese Anpassung kann in bestimmten Grenzen auch durch den Benutzer über eine Schnittstelle erfolgen, wodurch es diesem ermöglicht wird, auf die erzeugte Aerosol- bzw. Rauchmenge Einfluß zu nehmen. Der Heizstrom wird für eine voreingestellte Zeitperiode ("Heizperiode") geschaltet, welche typischerweise 1,0-1,8 Sekunden beträgt. Der Heizstrom wird den Verbunden 10 über die Steckzungen 8a und die Leiterbahnen 13 der Leiterplatte 11 zugeführt und bewirkt eine blitzartige Aufheizung der Verbunde 10 und des in den Dochten gespeicherten flüssigen Materials 17, woraufhin das flüssige Material 17 verdampft. Der Dampf wird in die Kammer 26 emittiert, wo er sich mit der durch die Luftdüse 29 einströmenden Luft mischt.

Die Anordnung und Dimensionierung der Luftdüse 29 bewirkt eine gleichmäßige und schnelle Überströmung der Verbunde 10. Hierdurch wird sichergestellt, daß der von den Verbunden 10 freigesetzte Dampf allseits annähernd gleiche Mischungsbedingungen vorfindet, und die Mischung von Dampf und Luft innig ist. Die Luft bewirkt eine Kühlung des Dampfes, so daß sich außerdem ein Kondensationsaerosol ausbilden kann, sofern das verdampfte flüssige Material 17 Substanzen mit hinreichend niedrigem Dampfdruck - sogenannte aerosolbildende Substanzen - enthält. Ein typisches Beispiel für solche aerosolbildenden Substanzen ist Glycerol.

Das in der Kammer 26 gebildete Dampf-Luft-Gemisch oder/und Kondensationsaerosol durchströmt schließlich im Ausführungsbeispiel noch einen Kühler 34, bevor es dem Benutzer über das Mundstück 4 zur Inhalation dargeboten wird (siehe Fig. 4g und Fig. 8). Der Kühler 34 kann beispielsweise aus einem porösen Füllmaterial, einem vliesartigen Fasermaterial oder aus einem offenzelligen Schaummaterial bestehen, dessen Poren vom gebildeten Dampf-Luft-Gemisch oder/und Kondensationsaerosol durchströmt werden. Der Kühler 34 kann auch mehrstufig ausgeführt sein, wobei die einzelnen Kühlerstufen unterschiedliche Eigenschaften aufweisen. Enthält das zu verdampfende Material Nikotin, kann es von Vorteil sein, das Kühlermaterial zumindest einer Kühlerstufe mit einem geeigneten Absorbens, beispielsweise mit Zitronensäure zu beschichten. Das Absorbens entzieht dem durchströmenden Kondensationsaerosol leichtflüchtige Nikotinfraktionen, welche ansonsten in der Mundhöhle und im Rachen niedergeschlagen würden, was weder pharmakokinetisch noch organoleptisch wünschenswert ist. Dem Kühlermaterial können ferner Aromastoffe wie Menthol zugefügt werden.

Geeignete vliesartige Fasermaterialien können beispielsweise von der Firma Freudenberg Vliesstoffe KG, www.freudenberg-filter.com, bezogen werden. Das unter der Bezeichnung Viledon®-Filtermatten vertriebene und aus Polyolefinfasern bestehende Material wird nach Kundenspezifikation angefertigt, wobei die Materialeigenschaften so abgestimmt werden können, daß das Endprodukt für die feinen Partikel des erzeugten Kondensationsaerosols weitestgehend durchlässig ist. Ein geeignetes Schaummaterial kann zum Beispiel von der Firma Dunlop Equipment, www.dunlop-equipment.com bezogen werden. Der angeführte Lieferant bietet Ni- und NiCr-Schaum unter der Produktbezeichnung Retimet® (Grade 80) mit einer Porosität von 90-95% und einem Porendurchmesser von etwa 300µm in Plattenform bis zu Dicken von 15mm an. Nach mündlicher Mitteilung von Firmenvertretern können aus technologischer Sicht auch noch etwas feinporigere Schäume hergestellt werden. Die Metallschäume können außerdem durch Walzen zusätzlich verdichtet werden. Die Platten können durch Laserschneiden oder Drahterodieren weiterverarbeitet werden. Ni-Schaum und insbesondere NiCr-Schaum zeichnen sich durch eine hohe Festigkeit sowie durch eine hohe Temperatur- und Oxidationsbeständigkeit aus. Diese Eigenschaften legen es nahe, die vergleichsweise teuren Metallschäume am Ende der Nutzungsdauer der Inhalatorkomponente 2 zu recyclen und wiederzuverwenden. Enthält das flüssige Material 17 Nikotin, sollte die Inhalatorkomponente 2 grundsätzlich nur gegen ein angemessenes Pfand an den Verbraucher abgegeben werden. Auf diese Weise wird sichergestellt, daß der Großteil der mit Nikotinrückständen kontaminierten Kühler 34, Schwämme 25a, 25b und Flüssigkeitsbehälter 18 umweltgerecht entsorgt und gegebenenfalls wiederaufbereitet wird.

Am Ende der Heizperiode deaktiviert der Schaltkreis 7 den Schalter 7a für ein paar Sekunden. Die Deaktivierung wird dem Benutzer zum Beispiel durch eine Leuchtdiode angezeigt und ist erforderlich, damit die Verbunde 10 abkühlen können, und die Dochte sich von neuem mit dem flüssigen Material 17 vollsaugen können. Der Flüssigkeitstransport wird durch die Kapillarität der Verbunde 10 bzw. deren Dochte bewirkt. Die Dochte saugen das flüssige Material 17 über die Verbund-Endabschnitte 10a, 10b aus dem ersten Kapillarspalt-Abschnitt 16a (siehe Fig. 4b/4f und Fig. 11). Die Dochte werden also von zwei Seiten infiltriert. Die Entnahme von flüssigen Material 17 aus dem ersten Kapillarspalt-Abschnitt 16a induziert im Kapillarspalt 16 einen Kapillardruck, welcher bis in den Flüssigkeitsbehälter 18 zurückwirkt. Der Kapillardruck hat zur Folge, daß flüssiges Material 17 aus dem Flüssigkeitsbehälter 18 über die schlitzförmige Versorgungsöffnung 20 in den zweiten Kapillarspalt-Abschnitt 16b nachströmt (siehe Pfeile in Fig. 4a). Von dort gelangt das flüssige Material 17 über den dritten Kapillarspalt-Abschnitt 16c in den ersten Kapillarspalt-Abschnitt 16a, wo es schließlich die entnommene Flüssigkeitsmenge ersetzt. Treten im Kapillarspalt-System 16 an einer oder mehreren Stellen - aus welchen Gründen auch immer - Störungen der Kapillarströmung auf, so wird sich in den meisten Fällen ein alternativer Pfad zur Umgehung der betroffenen Stellen finden.

Die aus dem Flüssigkeitsbehälter 18 entnommene Menge flüssigen Materials 17 wird im Zuge eines Druckausgleichs durch eine äquivalente Menge Luft ersetzt. Der Druckausgleich erfolgt über die Belüftungsnut 24 und die Belüftungsöffnung 23. Sobald die Verbunde 10 bzw. Dochte wieder vollständig mit dem flüssigen Material 17 infiltriert sind, steht der Inhalator für einen neuerlichen Verdampfungszyklus bereit.

In einer umgedrehten Gebrauchsstellung der Inhalatorkomponente 2 - das Mundstück 4 weist nach unten - geht die kapillare Kopplung zwischen dem Kapillarspalt 16 und dem flüssigen Material 17 im Flüssigkeitsbehälter 18 verloren, weil das im Flüssigkeitsbehälter 18 immer vorhandene Luftpolster 35 aufgrund des Auftriebs in jeder Lage stets nach oben steigt, also in der umgedrehten Gebrauchsstellung im Bereich der Versorgungsöffnung 20 zu liegen kommt. Ein Betrieb des Inhalators ist dennoch möglich, zumindest für eine gewisse Anzahl von Zügen bzw. Inhalationen, weil im ausgedehnten Kapillarspalt-System 16 ausreichend flüssiges Material 17 gepuffert ist. Erst wenn alle Kapillarspalt-Abschnitte 16a, 16b, 16c vollständig leer sind, drohen die Dochte auszutrocknen. Spätestens zu diesem Zeitpunkt ist es notwendig, die Inhalatorkomponente 2 wieder in eine normale Gebrauchsstellung zu drehen, so daß sich der Kapillarspalt 16 wieder mit dem flüssigen Material 17 füllen kann, welcher Vorgang übrigens nur wenige Sekunden in Anspruch nimmt.

Abschließend soll beispielhaft noch eine nikotinhaltige Zubereitung des flüssigen Materials 17 offenbart werden, welche in Prototypen verdampft wurde (siehe Tabelle 1). Das hierbei gebildete und verabreichte Kondensationsaerosol kam hinsichtlich der pharmakologischen, pharmakokinetischen sowie organoleptischen Wirkungen dem Rauch einer konventionellen Zigarette sehr nahe. Sämtliche aufgeführten Inhaltsstoffe finden sich auch im Zigarettenrauch wieder.

**Tabelle 1:**

| Stoff | CAS-Nummer | Massen-% |
|---|---|---|
| Wasser | 7732-18-5 | 52,88 |
| Ethanol | 64-17-5 | 4,14 |
| Glycerol (E422) | 56-81-5 | 40,04 |
| Nikotin | 54-11-5 | 1,33 |
| Milchsäure (E270) | 50-21-5 | 0,33 |
| Bernsteinsäure (E363) | 110-15-6 | 0,33 |
| Benzoesäure (E210) | 65-85-0 | 0,24 |
| Essigsäure (E260) | 64-19-7 | 0,71 |
| | Summe: | 100,00 |

Es sei noch darauf hingewiesen, daß die Erfindung selbstverständlich nicht auf einen oder mehrere flächige Verbunde 10 gemäß dem soeben beschriebenen Ausführungsbeispiel beschränkt ist. Die Verbunde können alternativ auch linien- bzw. fadenförmig ausgebildet sein. Die Verbunde können ferner in beliebiger Weise miteinander elektrisch verschaltet sein. Schließlich umfaßt die Erfindung auch Vorrichtungen, in welchen der Flüssigkeitsbehälter 18 vom Gehäuse 3 trennbar angeordnet ist, so daß der Flüssigkeitsbehälter 18, sobald er leer ist, durch einen neuen Flüssigkeitsbehälter ersetzt werden kann.

### Bezugszeichenliste

- 1: wiederverwendbares Inhalatorteil
- 2: auswechselbare Inhalatorkomponente
- 3: Gehäuse
- 3a: Gehäusewand
- 4: Mundstück
- 5: Hauptgehäuse
- 6: Batterie
- 7: elektrischer Schaltkreis
- 7a: Schalter
- 8a: Steckzungen
- 8b: Steckbuchsen
- 9a: Führungsnasen
- 9b: Führungsnuten
- 10: flächige Verbunde
- 10a, 10b: Verbund-Endabschnitte
- 11: Trägerplatte, Leiterplatte, Multilayer-Leiterplatte
- 11a: Leiterplatten-Vorderseite
- 11b: Leiterplatten-Rückseite
- 11c: Leiterplattenrand
- 12: Ausnehmung
- 13: Leiterbahnen
- 14: Oberteil
- 15: Aussparung
- 16: Kapillarspalt, Kapillarspalt-System
- 16a: erster Kapillarspalt-Abschnitt
- 16b: zweiter Kapillarspalt-Abschnitt
- 16c: dritter Kapillarspalt-Abschnitt
- 17: flüssiges Material
- 18: Flüssigkeitsbehälter
- 18a: Flüssigkeitsbehälterwand
- 19: Abstandshalter
- 20: Versorgungsöffnung
- 21: Fortsatz
- 22: Steg
- 23: Belüftungsöffnung
- 24: Belüftungsnut
- 25a, 25b: offenporige, saugfähige Schwämme
- 26: Kammer
- 27: U-förmiger Träger
- 27a, 27b: Winkelprofile
- 27c: Schenkel
- 28: Vertiefung
- 29: Luftdüse
- 30: Ansaugschnorchel
- 31: Loch
- 32: Strömungsdrossel
- 33: Plenumkammer
- 34: Kühler
- 35: Luftpolster

## Patentansprüche

1. Inhalatorkomponente für die Bildung eines Dampf-Luft-Gemisches oder/und Kondensationsaerosols durch Verdampfung eines flüssigen Materials (17) und gegebenenfalls Kondensation des gebildeten Dampfes, umfassend:
ein Gehäuse (3);
ein elektrisches Heizelement zur Verdampfung einer Portion des flüssigen Materials;
einen Docht mit einer Kapillarstruktur, welcher Docht mit dem Heizelement einen Verbund (10) bildet und das Heizelement selbsttätig mit dem flüssigen Material (17) versorgt;
eine Trägerplatte (11), vorzugsweise Leiterplatte, welche den Verbund (10) trägt und auf welcher das Heizelement elektrisch kontaktiert ist;
einen zumindest zum Teil durch die Trägerplatte (11) gebildeten Kapillarspalt (16) zur selbsttätigen Versorgung des Verbundes (10) mit dem flüssigen Material (17), indem ein Endabschnitt des Dochts in den Kapillarspalt (16) ragt,
**dadurch gekennzeichnet, daß** sowohl die Vorderseite (11a) als auch die Rückseite (11b) der Trägerplatte (11) zumindest abschnittsweise Begrenzungswände des Kapillarspalts (16) bilden.

2. Inhalatorkomponente **nach Anspruch 1, dadurch gekennzeichnet, daß** auch der Trägerplattenrand (11c) zumindest abschnittsweise eine Begrenzungswand des Kapillarspalts (16) bildet.

3. Inhalatorkomponente **nach Anspruch 2, dadurch gekennzeichnet, daß** der Kapillarspalt (16) die Trägerplatte (11) zumindest teilweise umschließt.

4. Inhalatorkomponente **nach einem der Ansprüche 1-3, dadurch gekennzeichnet, daß** über 50 Prozent der Trägerplatten-Oberfläche Begrenzungswände des Kapillarspalts (16) bilden.

5. Inhalatorkomponente **nach einem der Ansprüche 1-4, dadurch gekennzeichnet, daß** der Kapillarspalt (16) zumindest abschnittsweise durch die Trägerplatte (11) und eine benachbarte Wand (3a) des Gehäuses (3) gebildet wird.

6. Inhalatorkomponente **nach einem der Ansprüche 1-5** mit einem das flüssige Material (17) enthaltenden Flüssigkeitsbehälter (18), von welchem der Kapillarspalt (16) das flüssige Material (17) bezieht, **dadurch gekennzeichnet, daß** der Kapillarspalt (16) zumindest abschnittsweise durch die Trägerplatte (11) und eine benachbarte Wand (18a) des Flüssigkeitsbehälters (18) gebildet wird.

7. Inhalatorkomponente **nach Anspruch** 6, **dadurch gekennzeichnet, daß** der Kapillarspalt (16) über eine Versorgungsöffnung (20) in der Wand (18a) des Flüssigkeitsbehälters (18) mit dem flüssigen Material (17) im Flüssigkeitsbehälter (18) kommuniziert, indem die Wand (18a) des Flüssigkeitsbehälters (18) am Rand der Versorgungsöffnung (20) einen Fortsatz (21) bildet, an welchen Fortsatz (21) die Trägerplatte (11) mit ihrem Rand (11c) stößt.

8. Inhalatorkomponente **nach Anspruch 6, dadurch gekennzeichnet, daß** der Kapillarspalt (16) über eine Versorgungsöffnung (20) in der Wand (18a) des Flüssigkeitsbehälters (18) mit dem flüssigen Material (17) im Flüssigkeitsbehälter (18) kommuniziert, indem die Wand (18a) des Flüssigkeitsbehälters (18) am Rand der Versorgungsöffnung (20) einen Fortsatz (21) bildet, auf welchem Fortsatz (21) die Trägerplatte (11) mit ihrer Vorder- oder Rückseite (11a), (11b) aufliegt.

9. Inhalator, umfassend eine Inhalatorkomponente (2) **nach einem der Ansprüche 1-8.**

## Claims

1. Inhaler component for the formation of a vapour/ air mixture and/or condensation aerosol by evaporation of a liquid material (17) and, if appropriate, condensation of the vapour formed, comprising:
a housing (3);
an electrical heating element for evaporating a portion of the liquid material;
a wick with a capillary structure, which wick forms a composite structure (10) with the heating element and automatically supplies the heating element with the liquid material (17);
a carrier plate (11), preferably a printed circuit board, which carries the composite structure (10) and to which the heating element makes electrical contact;
a capillary gap (16) which is at least partly formed by the carrier plate (11) and which automatically supplies the composite structure (10) with the liquid material (17) by way of an end portion of the wick protruding into the capillary gap (16),
**characterized in that** both the front face (11a) and also the rear face (11b) of the carrier plate (11), at least in some areas, form boundary walls of the capillary gap (16).

2. Inhaler component according to Claim 1, **characterized in that** the edge (11c) of the carrier plate also forms, at least in some areas, a boundary wall of the capillary gap (16).

3. Inhaler component according to Claim 2, **characterized in that** the capillary gap (16) at least partially encloses the carrier plate (11).

4. Inhaler component according to one of Claims 1-3, **characterized in that** boundary walls of the capillary gap (16) form over 50 percent of the surface of the carrier plate.

5. Inhaler component according to one of Claims 1-4, **characterized in that** the capillary gap (16) is formed, at least in some areas, by the carrier plate (11) and an adjacent wall (3a) of the housing (3).

6. Inhaler component according to one of Claims 1-5 with a liquid container (18) which contains the liquid material (17) and from which the capillary gap (16) draws the liquid material (17), **characterized in that** the capillary gap (16), at least in some areas, is formed by the carrier plate (11) and an adjacent wall (18a) of the liquid container (18).

7. Inhaler component according to Claim 6, **characterized in that** the capillary gap (16) communicates with the liquid material (17) in the liquid container (18) via a supply opening (20) in the wall (18a) of the liquid container (18), by means of the wall (18a) of the liquid container (18) forming a shoulder (21) at the edge of the supply opening (20), on which shoulder (21) the carrier plate (11) abuts with its edge (11c).

8. Inhaler component according to Claim 6, **characterized in that** the capillary gap (16) communicates with the liquid material (17) in the liquid container (18) via a supply opening (20) in the wall (18a) of the liquid container (18), by means of the wall (18a) of the liquid container (18) forming a shoulder (21) at the edge of the supply opening (20), on which shoulder (21) the carrier plate (11) bears with its front face (11a) or rear face (11b).

9. Inhaler comprising an inhaler component (2) according to one of Claims 1-8.

## Revendications

1. Composant d'inhalateur pour la formation d'un mélange d'air et de vapeur et/ou d'un aérosol de condensation par évaporation d'une substance fluide (17) et éventuellement condensation de la vapeur formée, comprenant :
un boîtier (3) ;
un élément chauffant électrique pour l'évaporation d'une partie de la substance fluide ;
une mèche ayant une structure capillaire, laquelle mèche forme un ensemble (10) avec l'élément chauffant et alimente automatiquement l'élément chauffant en substance fluide (17) ;
une plaque de support (11), de préférence une carte à circuits imprimés, qui porte l'ensemble (10) et sur laquelle l'élément chauffant est mis en contact électrique ;
une fente capillaire (16) formée au moins en partie par la plaque de support (11) pour l'alimentation automatique de l'ensemble (10) en substance fluide (17), par le fait qu'une partie d'extrémité de la mèche pénètre dans la fente capillaire (16),
**caractérisé en ce qu'**à la fois le côté avant (11a) et le côté arrière (11b) de la plaque de support (11) forment au moins en partie des parois de limitation de la fente capillaire (16).

2. Composant d'inhalateur selon la revendication 1, **caractérisé en ce que** le bord de la plaque de support (11c) forme aussi au moins en partie une paroi de limitation de la fente capillaire (16).

3. Composant d'inhalateur selon la revendication de, **caractérisé en ce que** la fente capillaire (16) entoure au moins en partie la plaque de support (11).

4. Composant d'inhalateur selon l'une quelconque des revendications 1 à 3, **caractérisé en ce que** plus de 50 % de la surface de la plaque de support forment des parois de limitation de la fente capillaire (16).

5. Composant d'inhalateur selon l'une quelconque des revendications 1 à 4, **caractérisé en ce que** la fente capillaire (16) est formée au moins en partie par la plaque de support (11) et une paroi adjacente (3a) du boîtier (3).

6. Composant d'inhalateur selon l'une quelconque des revendications 1 à 5, comprenant un récipient de liquide (18) contenant la substance fluide (17), depuis lequel la fente capillaire (16) aspire la substance fluide (17), **caractérisé en ce que** la fente capillaire (16) est formée au moins en partie par la plaque de support (11) et une paroi adjacente (18a) du récipient de liquide (18).

7. Composant d'inhalateur selon la revendication 6, **caractérisé en ce que** la fente capillaire (16) communique par le biais d'une ouverture d'alimentation (20) dans la paroi (18a) du récipient de liquide (18) avec la substance fluide (17) dans le récipient de liquide (18), par le fait que la paroi (18a) du récipient de liquide (18) forme une saillie (21) au bord de l'ouverture d'alimentation (20), la plaque de support (11) venant en butée avec son bord (11c) contre ladite saillie (21).

8. Composant d'inhalateur selon la revendication 6, **caractérisé en ce que** la fente capillaire (16) communique par le biais d'une ouverture d'alimentation (20) dans la paroi (18a) du récipient de liquide (18) avec la substance fluide (17) dans le récipient de liquide (18), par le fait que la paroi (18a) du récipient de liquide (18) forme une saillie (21) au bord de l'ouverture d'alimentation (20), la plaque de support (11) s'appliquant avec son côté avant (11a) ou son côté arrière (11b) sur ladite saillie (21).

9. Inhalateur comprenant un composant d'inhalateur (2) selon l'une quelconque des revendications 1 à 8.
